# DEMANDE DE BREVET EUROPEEN

(11) **EP 0 801 064 A1**
(43) Date de publication de la demande: **15.10.1997**
(21) Numéro de dépôt: 97870047.4
(22) Date de dépôt: 09.04.1997
(51) Int. Cl.: C07D 295/08

(54) **Procédé de préparation de l'acide 2-(2-(4((4-chlorophényl) phénylméthyl)-1-pipérazinyl)éthoxy)-acétique et de ses sels**

(30) Priorité: 10.04.1996 BE 9600311
(71) Demandeur: U C B, S.A., 1050 Bruxelles (BE)
(72) Inventeur: Duchene, Guy, 1933 Sterrebeek (BE); Zimmermann, Vincent, 1050 Bruxelles (BE); Bodson, Guy, 6730 Bellefontaine (BE)

(57) **Abrégé**

Procédé de préparation de l'acide 2-[2-[4-[(4-chlorophényl)phénylméthyl]-1-pipérazinyl]éthoxy]-acétique dans lequel on fait réagir la 1-[(4-chlorophényl)phénylméthyl]-pipérazine avec l'acide 2-chloroéthoxyacétique, en présence d'un accepteur d'acide, dans un solvant inerte. Le cas échéant, l'acide 2-[2-[4-[(4-chlorophényl)-phénylméthyl]-1-pipérazinyl]éthoxy]-acétique est transformé en l'un de ses sels.

## Description

La présente invention se rapporte à un nouveau procédé de préparation de l'acide 2- [2-[4-[(4-chlorophényl)phénylméthyl]-1-pipérazinyl]éthoxy]-acétique et de ses sels.

L'acide 2-[2-[4-[(4-chlorophényl)phénylméthyl]-1-pipérazinyl]éthoxy]-acétique, également connu sous le nom générique de cétirizine, a été introduit sous la forme de son dichlorhydrate comme nouveau médicament pour le traitement des syndromes allergiques, comme la rhinite allergique chronique et aiguë, la conjonctivite allergique, le prurit, l'urticaire, etc. Dans son application thérapeutique, ce produit s'est avéré remarquablement dépourvu d'effets secondaires sur le système nerveux central tels que la somnolence, la performance mentale atténuée, etc. (cf. D.P. TASHKIN et al., Annals of Allergy, Part II, 59,(1987),49-52, ainsi que F.M. GENGO et al., Annals of Allergy, Part II, 59, (1987),53-57).

Le brevet européen n° 58146, au nom de la demanderesse, décrit la synthèse de l'acide 2-[2-[4-[(4-chlorophényl)phénylméthyl)-1-pipérazinyl]éthoxy]-acétique par réaction de la 1-[(4-chlorophényl)phénylméthyl]-pipérazine avec le 2-chloroéthoxyacétate de méthyle pour former le 2-[2-[4-[(4-chlorophényl)phénylméthyl]-1-pipérazinyl]éthoxy]-acétate de méthyle. Cet ester méthylique est ensuite soumis à une hydrolyse avec l'hydroxyde de potassium pour former le sel potassique que l'on transforme facilement en acide 2-[2-[4-[(4-chlorophényl)phénylméthyl]-1-pipérazinyl]éthoxy]-acétique et en son dichlorhydrate.

L'inconvénient principal de ce procédé est que le rendement global en dichlorhydrate de l'acide 2-[2-[4-[(4-chlorophényl)phénylméthyl]-1-pipérazinyl]éthoxy]-acétique n'est que de 10,6% par rapport à la quantité de 1-[(4-chlorophényl)phénylméthyl]-pipérazine mise en oeuvre.

Le brevet britannique n° 2225321, également au nom de la demanderesse, décrit la synthèse de l'acide 2-[2-[4-[(4-chlorophényl)phénylméthyl]-1-pipérazinyl]éthoxy]-acétique et de son dichlorhydrate par réaction de la 1-[(4-chlorophényl)phénylméthyl]-pipérazine avec le 2-chloroéthoxyacétonitrile pour former le 2-[2-[4-[(4-chlorophényl)phénylméthyl]-1-pipérazinyl]éthoxy]acétonitrile. Ce dernier est ensuite soumis à une hydrolyse en milieu acide ou basique pour donner l'acide 2-[2-[4-[(4chlorophényl)phénylméthyl]-1-pipérazinyl]éthoxy]-acétique qui peut, le cas échéant, être transformé en son dichlorhydrate.

L'inconvénient majeur de ce procédé réside dans le fait que la préparation du 2-chloroéthoxyacétonitrile pose des problèmes de toxicité graves sur le plan industriel. En effet, dans le brevet britannique 2225321, ce composé est préparé par la méthode décrite par E.J. SALMI et al., Suomen Kemistilehti, 17B, (1944), 17-19 (Chem. Abstr. 40, (1946), 6491) qui consiste à préparer dans une première étape le (2-chloroéthyl)chlorométhyl éther et à traiter ensuite cet éther avec du cyanure de cuivre (I), pour former le 2-chloroéthoxyacétonitrile. Ce mode de préparation du 2-chloroéthoxyacétonitrile est difficilement utilisable industriellement car il fait intervenir le (2-chloroéthyl)chlorométhyl éther et du cyanure de cuivre (I) qui sont des produits fortement toxiques, nécessitant des mesures de protection particulières.

Le brevet britannique n° 2225320, également au nom de la demanderesse, décrit encore une autre synthèse de l'acide 2-[2-[4-[(4-chlorophényl)phénylméthyl]-1-pipérazinyl]éthoxy]-acétique et de son dichlorhydrate par réaction de la 1-[(4-chlorophényl)phénylméthyl]-pipérazine avec le 2-chloroéthanol pour donner le 2-[4-[(4-chlorophényl)phénylméthyl]-1-pipérazinyl]éthanol que l'on fait ensuite réagir avec le chloroacétate de sodium, en présence de tert-butylate de sodium. Le sel de sodium ainsi obtenu est converti en acide 2-[2-[4-[(4-chlorophényl)phénylméthyl]-1-pipérazinyl]éthoxy]-acétique et en son dichlorhydrate. Ce procédé permet d'obtenir un rendement global en dichlorhydrate de l'acide 2-[2-[4-[(4-chlorophényl)phénylméthyl]-1-pipérazinyl]éthoxy]-acétique de 44% par rapport à la quantité de l-[(4-chlorophényl)phénylméthyl]-pipérazine mise en oeuvre.

Selon la présente invention, la demanderesse vient de découvrir que l'acide 2-[2-[4-[(4-chlorophényl)phénylméthyl]-1-pipérazinyl]éthoxy]-acétique peut-être préparé par un procédé caractérisé en ce que l'on fait réagir la 1-[(4-chlorophényl)phénylméthyl]-pipérazine avec l'acide 2-chloroéthoxyacétique, en présence d'au moins un accepteur d'acide, dans un solvant inerte.

Ce procédé de synthèse présente de nombreux avantages par rapport aux procédés connus:
a. ce procédé ne fait pas intervenir d'intermédiaires ou de réactifs hautement toxiques;
b. ce procédé permet d'obtenir la cétirizine en une seule étape, au départ de la 1-[(4-chlorophényl)phénylméthyl]-pipérazine et de l'acide 2-chloroéthoxyacétique. En effet, si l'on compare ce procédé au procédé décrit dans le brevet européen n° 58146, ce dernier nécessite tout d'abord de préparer le 2-chloroéthoxyacétate de méthyle à partir de l'acide 2-chloroéthoxyacétique. Ensuite, on fait réagir le 2-chloroéthoxyacétate de méthyle avec la 1-[(4-chlorophényl)phénylméthyl]-pipérazine pour former le 2-[2-[4-[(4-chlorophényl)phénylméthyl]-1-pipérazinyl]éthoxy]-acétate de méthyle, qui doit encore subir une étape d'hydrolyse pour fournir l'acide 2-[2-[4-[(4-chlorophényl)phénylméthyl]-1-pipérazinyl]éthoxy]-acétique. Ce procédé comporte donc trois étapes. Par rapport au procédé décrit dans le brevet européen n° 58146, le procédé selon la présente invention permet d'économiser deux étapes dans la production de l'acide 2-[2-[4-[(4-chlorophényl)phénylméthyl]-1-pipérazinyl]éthoxy]-acétique, ce qui représente un avantage considérable pour la production à l'échelle industrielle;
c. l'étape unique de ce procédé est facilement mise en oeuvre à l'échelle industrielle. En particulier, elle ne nécessite qu'une mise au point de routine lors de l'addition des réactifs.

Selon l'invention, on prépare l'acide 2-[2-[4-[(4-chlorophényl)phénylméthyl]-1-pipérazinyl]éthoxy]-acétique par réaction de la 1-[(4-chlorophényl)phénylméthyl]-pipérazine avec l'acide 2-chloroéthoxyacétique en présence d'au moins un accepteur d'acide, dans un solvant inerte.

Parmi les accepteurs d'acides utilisables selon l'invention, on peut citer les bases minérales (par exemple, les carbonates de métaux alcalins, les hydroxydes de métaux alcalins, etc.) ou les bases organiques (par exemple les amines tertiaires telles que la triéthylamine, l'éthyldiisopropylamine, la N-éthylmorpholine ou la 2,4,6-triméthylpyridine). La quantité d'accepteur d'acide utilisée est telle qu'il y a entre 0,1 et 5,0 moles, de préférence entre 0,8 et 2,0 moles d'accepteur d'acide pour une mole d'acide 2-chloroéthoxyacétique engagé.

Parmi les solvants inertes utilisables selon l'invention, on peut citer les cétones aliphatiques (par exemple, la diisobutylcétone, la méthyléthylcétone), les hydrocarbures aromatiques (par exemple, le toluène ou le xylène).

Cette réaction est généralement effectuée par chauffage entre 80°C et 150°C, pendant plusieurs heures.

De préférence, la 1-[(4-chlorophényl)phénylméthyl]-pipérazine et l'acide 2-chloroéthoxyacétique sont utilisés dans des proportions molaires comprises entre 3:1 et 1:3.

Eventuellement, pour accélérer la réaction, cette dernière peut être effectuée en présence d'une faible quantité d'iodure de métal alcalin, dans un rapport molaire pouvant varier entre 0,5 et 5,0% d'iodure de métal alcalin pour une mole d'acide 2-chloroéthoxyacétique utilisé.

En fin de réaction, les produits secondaires et les sels inorganiques formés au cours de la réaction sont séparés par des méthodes traditionnelles.

Le cas échéant, l'acide 2-[2-[4-[(4-chlorophényl)phénylméthyl]-1-pipérazinyl]éthoxy]-acétique peut être converti en ses sels. Par sels, on entend non seulement les sels d'addition avec des acides organiques ou inorganiques, tels que les acides acétique, citrique, succinique, ascorbique, hydrochlorique, hydrobromique, sulfurique et phosphorique, mais également leurs sels métalliques (par exemple de sodium ou de potassium), d'ammonium, ainsi que les sels d'addition avec des amines et des acides aminés. L'exemple qui suit illustre l'invention sans toutefois la limiter.

### EXEMPLE Préparation de l'acide 2-[2-[4-[(4-chlorophényl)phénylméthyl]-1-pipérazinyl]éthoxy]-acétique et de son dichlorhydrate.

Dans un réacteur muni d'un agitateur mécanique, d'un réfrigérant et d'un thermomètre, on introduit successivement 100 ml de toluène, 100 g (0,348 mole) de l-[(4-chlorophényl)phénylméthyl]-pipérazine, 60.4 g (0,435 mole) d'acide 2-chloroéthoxyacétique, 57.6 g (0,417 mole) de carbonate de potassium et 1,4 g (0,008 mole) d'iodure de potassium. On chauffe le mélange sous agitation pendant 10 heures à 110°C. On refroidit ensuite le mélange réactionnel auquel on ajoute de l'eau. Le pH de la phase aqueuse est ajusté à 4,5-5,0. La phase organique est décantée. La phase aqueuse est évaporée et le résidu est repris dans du toluène que l'on porte à ébullition. La suspension ainsi obtenue est filtrée à chaud, puis évaporée. Le résidu est alors dissout dans la méthyl éthyl cétone. Cette solution est soumise à un courant d'acide chlorhydrique gazeux et est ensuite refroidie à 0°C. Le solide qui s'est formé est alors filtré et séché sous vide. Ce solide contient le dichlorhydrate de l'acide 2-[2-[4-[(4-chlorophényl)phénylméthyl]-1-pipérazinyl]éthoxy]-acétique.

## Revendications

1. Procédé de préparation de l'acide 2-[2-[4-[(4-chlorophényl)phénylméthyl]-1-pipérazinyl]éthoxy]-acétique, caractérisé en ce que l'on fait réagir la 1-[(4-chlorophényl)phénylméthyl]-pipérazine avec l'acide 2-chloroéthoxyacétique, en présence d'un accepteur d'acide, dans un solvant inerte.

2. Procédé de préparation selon la revendication 1, caractérisé en ce que l'acide 2-[2-[4-[(4-chlorophényl)phénylméthyl]-1-pipérazinyl]éthoxy]-acétique est ensuite converti en son dichlorhydrate.

3. Procédé selon l'une quelconque des revendications 1 et 2, caractérisé en ce que la réaction est effectuée à une température comprise entre 80°C et 150°C.

4. Procédé selon l'une quelconque des revendications 1 à 3, caractérisé en ce que le solvant inerte est choisi parmi les cétones aliphatiques et les hydrocarbures aromatiques.

5. Procédé selon l'une quelconque des revendications 1 à 4, caractérisé en ce que l'accepteur d'acide est choisi parmi le groupe constitué par les carbonates de métaux alcalins et les hydroxydes de métaux alcalins.

6. Procédé selon l'une quelconque des revendications 1 à 5, caractérisé en ce que l'accepteur d'acide est choisi parmi le groupe constitué par la triéthylamine, l'éthyldiisopropylamine, la N-éthylmorpholine ou la 2,4,6triméthylpyridine.

7. Procédé selon l'une quelconque des revendications 1 à 6, caractérisé en ce que la l-[(4-chlorophényl)phénylméthyl]-pipérazine et l'acide 2-chloroéthoxyacétique sont utilisés dans des proportions molaires comprises entre 3:1 et 1:3.

8. Procédé selon l'une quelconque des revendications 1 à 7, caractérisé en ce que la réaction est effectuée en présence d'iodure de métal alcalin.
